# EUROPEAN PATENT APPLICATION

(11) **EP 3 246 092 A1**
(43) Date of publication of application: **22.11.2017**
(21) Application number: 16170350.9
(22) Date of filing: 19.05.2016
(51) Int. Cl.: B01J 37/10, B01J 23/00, B01J 23/28, B01J 27/057, C07C 51/215

(54) **TREATMENT OF A MIXED METAL OXIDE CATALYST CONTAINING MOLYBDENUM, VANADIUM, NIOBIUM AND OPTIONALLY TELLURIUM**

(71) Applicant: Shell Internationale Research Maatschappij B.V., 2596 HR Den Haag (NL)
(72) Inventor: Roelofszen, Dennis Petrus Maria, 1031 HW Amsterdam (NL); Van Rossum, Guus, 1031 HW Amsterdam (NL); Schoonebeek, Ronald Jan, 1031 HW Amsterdam (NL); Verhaak, Michael Johannes Franciscus Maria, 1031 HW Amsterdam (NL)
(74) Representative: Matthezing, Robert Maarten

(57) **Abstract**

The invention relates to a process for treatment of a mixed metal oxide catalyst containing molybdenum, vanadium, niobium and optionally tellurium, comprising contacting a stream comprising water with the catalyst, wherein the catalyst is a fresh catalyst and the stream comprising water comprises substantially no alkane containing 2 to 6 carbon atoms and substantially no alkene containing 2 to 6 carbon atoms.

## Description

### Field of the invention

The present invention relates to a process for treatment of a mixed metal oxide catalyst containing molybdenum (Mo), vanadium (V), niobium (Nb) and optionally tellurium (Te).

### Background of the invention

Mixed metal oxide catalysts containing molybdenum, vanadium, niobium and optionally tellurium may be used in a variety of chemical processes. For example, such catalyst may be used to oxidatively dehydrogenate alkanes, such as alkanes containing 2 to 6 carbon atoms, for example ethane or propane resulting in ethylene and propylene, respectively, in an oxidative dehydrogenation (oxydehydrogenation; ODH) process. Examples of alkane ODH processes, including catalysts and other process conditions, are for example disclosed in US7091377, WO2003064035, US20040147393, WO2010096909 and US20100256432. Mixed metal oxide catalysts containing molybdenum, vanadium, niobium and optionally tellurium as the metals, can be used as such oxydehydrogenation catalysts. Such catalysts may also be used in the direct oxidation of alkenes to carboxylic acids, such as in the oxidation of alkenes containing 2 to 6 carbon atoms, for example ethylene or propylene resulting in acetic acid and acrylic acid, respectively.

It is desired to provide a process for treating fresh catalysts containing Mo, V, Nb and optionally Te, which have not been used as a catalyst in a chemical process before, such as to improve its performance, including its activity and/or selectivity.

Therefore, it is an objective of the present invention to provide a process for treatment of a mixed metal oxide catalyst containing molybdenum, vanadium, niobium and optionally tellurium, which catalyst is a fresh catalyst, which treatment process may result in an improvement in catalyst performance, in particular an improvement of the activity and/or selectivity of the catalyst, in particular an improvement of the activity and/or selectivity in an alkane ODH and/or alkene oxidation process wherein such catalyst may be used.

### Summary of the invention

Surprisingly it was found that such process for treatment of a mixed metal oxide catalyst containing molybdenum, vanadium, niobium and optionally tellurium may be a process which comprises contacting a stream comprising water with the fresh catalyst, in which case the catalyst performance, including activity and/or selectivity of the catalyst, may be improved.

Accordingly, the present invention relates to a process for treatment of a mixed metal oxide catalyst containing molybdenum, vanadium, niobium and optionally tellurium, comprising contacting a stream comprising water with the catalyst, wherein the catalyst is a fresh catalyst and the stream comprising water comprises substantially no alkane containing 2 to 6 carbon atoms and substantially no alkene containing 2 to 6 carbon atoms.

One important advantage of treating a catalyst such that it remains active and/or stable over time, is that there is no need for a frequent catalyst replacement (for example every few weeks or months) which would come at a high cost, in terms of catalyst consumption cost and downtime of a reactor.

### Detailed description of the invention

While the process of the present invention and a stream or catalyst used in said process are described in terms of "comprising", "containing" or "including" one or more various described steps or components, they can also "consist essentially of" or "consist of" said one or more various described steps or components.

In the context of the present invention, in a case where a stream or catalyst comprises two or more components, these components are to be selected in an overall amount not to exceed 100 vol.% or 100 wt.%.

Within the present specification, by "substantially no" in relation to the amount of a specific component, for example in a stream, it is meant an amount which is at most 1,000, preferably at most 500, more preferably at most 100, more preferably at most 50, more preferably at most 30, more preferably at most 20, and most preferably at most 10 ppmv (parts per million by volume) of the component in question, based on the total amount (i.e. volume) of all components, for example all components of said stream.

In the process of the present invention, a stream comprising water is contacted with the fresh catalyst. Preferably, said process comprises feeding the stream comprising water to a reactor, followed by contacting the stream comprising water with the catalyst in the reactor. In the latter case, the reactor contains the fresh catalyst. Further, during said feeding and contacting, the stream comprising water comprises substantially no alkane containing 2 to 6 carbon atoms and substantially no alkene containing 2 to 6 carbon atoms. Furthermore, during the process of the present invention, which may be carried out in a reactor, substantially no alkane containing 2 to 6 carbon atoms is present and substantially no alkene containing 2 to 6 carbon atoms is present. This implies that substantially no alkane containing 2 to 6 carbon atoms and substantially no alkene containing 2 to 6 carbon atoms are fed during said process, for example to a reactor containing the fresh catalyst. During the process of the present invention, substantially no alkane containing 2 to 6 carbon atoms and substantially no alkene containing 2 to 6 carbon atoms are fed, for example to a reactor containing the catalyst. However, during said process, other components may be fed. Such other components suitably comprise methane, an inert gas or oxygen (O₂) or any combination of two or more of these. Upon feeding one or more of said components, it or they is or are contacted with the catalyst, in addition to the water.

Preferably, during the process of the present invention, which may be carried out in a reactor, substantially no oxygen is present. This implies that substantially no oxygen is fed during said process, for example to a reactor containing the fresh catalyst.

In the process of the present invention, a stream comprising water may be fed for example to a reactor. In addition, methane, an inert gas or oxygen or any combination of two or more of these may be fed. Said components may be fed together or separately. That is to say, one or more feed streams, suitably gas streams, comprising one or more of said components may be fed. For example, one feed stream comprising oxygen and water may be fed. Alternatively, two or more feed streams, suitably gas streams, may be fed, which feed streams may form a combined stream inside a reactor. For example, one feed stream comprising water and another feed stream comprising oxygen may be fed separately.

Further, irrespective of whether (i) water and (ii) methane, an inert gas or oxygen or any combination of two or more of these are fed together or separately in the same or different feed streams as described above, said components are suitably fed simultaneously (at the same time).

In the process of the present invention, (i) water and (ii) optionally methane, an inert gas or oxygen or any combination of two or more of these is or are suitably fed in the gas phase. Further, in the process of the present invention, water may be fed in the gas phase or in the liquid phase, suitably in the gas phase. In particular, water may be fed as a steam which is in the gas phase which may be (i) water in the gas phase which is formed when water boils or (ii) water in the gas phase which is formed by passing a dry (water-unsaturated) stream containing oxygen and/or an inert gas through liquid water. Further, in particular, water may be fed as a wet steam which is a mist or aerosol of water droplets formed as water vapor condenses. In case water is fed in the liquid phase, the water suitably evaporates under the conditions inside a reactor.

In a case wherein during the process of the present invention oxygen is fed, the molar ratio of water as fed to oxygen as fed is suitably smaller than 1:1. As mentioned above, the water and oxygen may be fed in the same feed stream or in different feed streams. Furthermore, each of the components may be fed in two or more feed streams. Thus, the above-mentioned "molar ratio of water as fed to oxygen as fed" refers to the total molar amount of water as fed relative to the total molar amount of oxygen as fed.

In a case wherein during the process of the present invention oxygen is fed, the molar ratio of water as fed to oxygen as fed is preferably of from 0.01:1 to smaller than 1:1, more preferably 0.1:1 to 0.8:1, most preferably 0.2:1 to 0.6:1. Said molar ratio may be at least 0.01, or at least 0.05:1, or at least 0.1:1, or at least 0.15:1, or at least 0.2:1, or at least 0.25:1, or at least 0.3:1, or at least 0.35:1, or at least 0.4:1. Further, said molar ratio may be at most smaller than 1:1 and may be at most 0.9:1, or at most 0.8:1, or at most 0.75:1, or at most 0.7:1, or at most 0.65:1, or at most 0.6:1.

Further, in a case wherein during the process of the present invention oxygen is fed, the total amount of water and oxygen as fed, based on the total amount of components as fed, is suitably of from 10 to 80 vol%, more suitably 15 to 70 vol.%, most suitably 20 to 60 vol.%. Said total amount may be at least 5 vol.%, or at least 10 vol.%, or at least 15 vol.%, or at least 20 vol.%, or at least 25 vol.%. Further, said total amount may be at most 90 vol.%, or at most 80 vol.%, or at most 70 vol.%, or at most 60 vol.%, or at most 50 vol.%, or at most 40 vol.%, or at most 35 vol.%, or at most 30 vol.%.

As mentioned above, during the process of the present invention an inert gas and/or oxygen may be fed. Said inert gas may be selected from the group consisting of the noble gases and nitrogen (N₂). Preferably, the inert gas is nitrogen or argon, more preferably nitrogen. Said oxygen may originate from any source, such as for example air.

Preferably, in the present invention, the mixed metal oxide catalyst containing molybdenum, vanadium, niobium and optionally tellurium is a heterogeneous catalyst.

In the present invention, the catalyst is a mixed metal oxide catalyst containing molybdenum, vanadium, niobium and optionally tellurium as the metals, which catalyst may have the following formula:

Mo₁VₐTe_{b}Nb_{c}Oₙ

wherein:
a, b, c and n represent the ratio of the molar amount of the element in question to the molar amount of molybdenum (Mo);
a (for V) is from 0.01 to 1, preferably 0.05 to 0.60, more preferably 0.10 to 0.40, more preferably 0.20 to 0.35, most preferably 0.25 to 0.30;
b (for Te) is 0 or from >0 to 1, preferably 0.01 to 0.40, more preferably 0.05 to 0.30, more preferably 0.05 to 0.20, most preferably 0.09 to 0.15;
c (for Nb) is from >0 to 1, preferably 0.01 to 0.40, more preferably 0.05 to 0.30, more preferably 0.10 to 0.25, most preferably 0.14 to 0.20; and
n (for O) is a number which is determined by the valency and frequency of elements other than oxygen.

Examples of said mixed metal oxide catalysts are for example disclosed in above-mentioned US7091377, WO2003064035, US20040147393, WO2010096909 and US20100256432, the disclosures of which are herein incorporated by reference.

Preferably, in the present process, the temperature is of from 200 to 500 °C. More preferably, said temperature is of from 250 to 500 °C, most preferably of from 300 to 500 °C.

Preferably, said temperature is at least 200 °C, more preferably at least 250 °C, more preferably at least 280 °C, more preferably at least 300 °C, more preferably at least 320 °C, more preferably at least 340 °C, most preferably at least 350 °C.

Further, preferably, said temperature is at most 500 °C, more preferably at most 480 °C, more preferably at most 460 °C, more preferably at most 440 °C, more preferably at most 420 °C, most preferably at most 400 °C.

Still further, in the present process, typical pressures are 0.1-20 bara (i.e. "bar absolute"). Further, in a preferred embodiment, said pressure is of from 0.1 to 15 bara, more preferably of from 0.5 to 10 bara, most preferably of from 1 to 5 bara.

The time period for contacting the treatment gas stream with the catalyst may vary within wide ranges and may be of from 10 minutes to 10 hours, more suitably of from 30 minutes to 5 hours.

The treatment of the present process is applied to a fresh catalyst containing molybdenum, vanadium, niobium and optionally tellurium. Within the present specification, a "fresh catalyst" means a catalyst which has not been used as a catalyst in a chemical process before. The fresh catalyst is, however, suitable to be used as a catalyst in a chemical process, which means that it is a final catalyst obtained as the product in a catalyst preparation process, and not any intermediate catalyst or catalyst precursor. The present treatment process may be applied in order to improve catalyst performance, including activity and/or selectivity, of such fresh catalyst. Preferably, said fresh catalyst is intended to be used in the oxidative dehydrogenation of an alkane containing 2 to 6 carbon atoms and/or the oxidation of an alkene containing 2 to 6 carbon atoms.

As discussed above, the fresh catalyst may be intended to be used as a catalyst in the oxidative dehydrogenation of an alkane containing 2 to 6 carbon atoms and/or the oxidation of an alkene containing 2 to 6 carbon atoms. Preferably, in said alkane oxidative dehydrogenation process, the alkane containing 2 to 6 carbon atoms is a linear alkane in which case said alkane may be selected from the group consisting of ethane, propane, butane, pentane and hexane. Further, preferably, said alkane contains 2 to 4 carbon atoms and is selected from the group consisting of ethane, propane and butane. More preferably, said alkane is ethane or propane. Most preferably, said alkane is ethane. Further, preferably, in said alkene oxidation process, the alkene containing 2 to 6 carbon atoms is a linear alkene in which case said alkene may be selected from the group consisting of ethylene, propylene, butene, pentene and hexene. Further, preferably, said alkene contains 2 to 4 carbon atoms and is selected from the group consisting of ethylene, propylene and butene. More preferably, said alkene is ethylene or propylene.

The product of said alkane oxidative dehydrogenation process may comprise the dehydrogenated equivalent of the alkane, that is to say the corresponding alkene. For example, in the case of ethane such product may comprise ethylene, in the case of propane such product may comprise propylene, and so on. Such dehydrogenated equivalent of the alkane is initially formed in said alkane oxidative dehydrogenation process. However, in said same process, said dehydrogenated equivalent may be further oxidized under the same conditions into the corresponding carboxylic acid which may or may not contain one or more unsaturated double carbon-carbon bonds. As mentioned above, it is preferred that the alkane containing 2 to 6 carbon atoms is ethane or propane. In the case of ethane, the product of said alkane oxidative dehydrogenation process may comprise ethylene and/or acetic acid, preferably ethylene. Further, in the case of propane, the product of said alkane oxidative dehydrogenation process may comprise propylene and/or acrylic acid, preferably acrylic acid.

The product of said alkene oxidation process comprises the oxidized equivalent of the alkene. Preferably, said oxidized equivalent of the alkene is the corresponding carboxylic acid. Said carboxylic acid may or may not contain one or more unsaturated double carbon-carbon bonds. As mentioned above, it is preferred that the alkene containing 2 to 6 carbon atoms is ethylene or propylene. In the case of ethylene, the product of said alkene oxidation process may comprise acetic acid. Further, in the case of propylene, the product of said alkene oxidation process may comprise acrylic acid.

## Claims

1. Process for treatment of a mixed metal oxide catalyst containing molybdenum, vanadium, niobium and optionally tellurium, comprising contacting a stream comprising water with the catalyst, wherein the catalyst is a fresh catalyst and the stream comprising water comprises substantially no alkane containing 2 to 6 carbon atoms and substantially no alkene containing 2 to 6 carbon atoms.

2. Process according to claim 1, comprising feeding the stream comprising water to a reactor, followed by contacting the stream comprising water with the catalyst in the reactor.

3. Process according to claim 1 or 2, wherein during the process of the present invention substantially no alkane containing 2 to 6 carbon atoms is present and substantially no alkene containing 2 to 6 carbon atoms is present.

4. Process according to any one of the preceding claims, wherein the fresh catalyst is intended to be used as a catalyst in the oxidative dehydrogenation of an alkane containing 2 to 6 carbon atoms and/or the oxidation of an alkene containing 2 to 6 carbon atoms.
